# EUROPEAN PATENT APPLICATION

(11) **EP 4 477 252 A1**
(43) Date of publication of application: **18.12.2024**
(21) Application number: 23834567.2
(22) Date of filing: 05.06.2023
(51) Int. Cl.: A61M 60/81, A61M 60/126

(54) **DRIVING DEVICE AND BLOOD PUMP**

(30) Priority: 08.07.2022 CN 202210801382
(71) Applicant: Shenzhen Core Medical Technology Co., Ltd., Shenzhen, Guangdong 518000 (CN)
(72) Inventor: XIE, Duanqing, Shenzhen, Guangdong 518000 (CN); YU, Shunzhou, Shenzhen, Guangdong 518000 (CN)
(74) Representative: Appleyard Lees IP LLP
(86) International application number: PCT/CN2023/098354
(87) International publication number: WO 2024/007793

(57) **Abstract**

A driving device (10) and a blood pump (1). The driving device (10) comprises a housing (100), a rotating shaft (200), a first rotor (300), a first shaft sleeve (400), a second shaft sleeve (500), and a limiting component (600). The first rotor (300) is fixedly connected to one end of the rotating shaft (200). The first rotor (300) has a ball head part (310). The first shaft sleeve (400) is provided with a groove (410) with a concave spherical groove wall (412). The ball head part (310) is movably arranged in the groove (410). The rotating shaft (200) is rotatably arranged on the second shaft sleeve (500) in a penetrating manner. The limiting component (600) is fixedly connected to the rotating shaft (200). The limiting component (600) is located between the second shaft sleeve (500) and the first rotor (300). The limiting component (600) can abut against the second shaft sleeve (500). The blood pump (1) comprises the driving device (10) and an impeller (20).

## Description

### CROSS-REFERENCE TO RELATED APPLICATIONS

This application claims priority of Chinese Patent Application No. CN 202210801382.2, filed on July 8, 2022, the entire content of which is incorporated herein by reference in its entirety.

### TECHNICAL FIELD

The present application relates to the technical field of medical devices, and in particular to a driving device and a blood pump.

### BACKGROUND

Blood pump is designed to be inserted percutaneously into a patient's blood vessel, such as an artery or a vein in a thigh or an axilla, and can be advanced into the patient's heart to function as a left ventricular assist device or a right ventricular assist device.

The blood pump generally includes a driving device and an impeller, and the impeller is connected to a driving shaft of the driving device. In order to achieve stable rotation of the driving shaft, it is usually necessary to add a component for limiting the driving shaft, which makes the structure of the driving device complicated and increases the difficulty of assembling the driving device.

### SUMMARY

Accordingly, the present application provides a driving device and a blood pump which are easy to assemble.

An embodiment of a first aspect of the present application provides a driving device, including:
a housing;
a rotating shaft configured to be connected to an impeller, the rotating shaft being rotatably mounted to the housing;
a first rotor fixedly connected an end of the rotating shaft, the first rotor including a spherical head portion protruding along an axis of the rotating shaft;
a first shaft sleeve mounted to the housing, the first shaft sleeve being provided with a groove having a concave spherical groove wall, wherein the spherical head portion is movably provided in the groove, and the spherical head portion is capable of abutting against the spherical groove wall;
a second shaft sleeve mounted to the housing, wherein the rotating shaft rotatably extends through the second shaft sleeve, and the first rotor is located between the first shaft sleeve and the second shaft sleeve; and
a limiting member fixedly connected to the rotating shaft, the limiting member being located between the second shaft sleeve and the first rotor, and the limiting member being capable of abutting against the second shaft sleeve.

An embodiment of a second aspect of the present application provides a blood pump, which includes an impeller and the driving device as described in the first aspect. The rotating shaft is connected to the impeller, and the rotating shaft is capable of driving the impeller to rotate.

Details of one or more embodiments of the present application are set forth in the following drawings and descriptions. Other features, objects and advantages of the present application will become apparent with reference to the specification, drawings, and claims.

### BRIEF DESCRIPTION OF THE DRAWINGS

In order to illustrate the embodiments of the present application more clearly, the drawings used in the embodiments will be described briefly. Apparently, the following described drawings are merely for the embodiments of the present application, and other drawings can be derived by those of ordinary skill in the art without any creative effort.
FIG. 1 is a perspective view of a blood pump according to an embodiment of the present application.
FIG. 2 is a cross-sectional view of the blood pump shown in FIG. 1 with a cannula, an impeller, and a part of a catheter omitted.
FIG. 3 is a cross-sectional view of a shaft, a pushing stop ring, a first rotor, a second rotor, a first shaft sleeve, and a second shaft sleeve of the blood pump of FIG. 1 assembled together.
FIG. 4 is an enlarged view of a portion A shown in FIG. 2.
FIG. 5 is a perspective view of a first flywheel of the first rotor shown in FIG. 2.
FIG. 6 is a cross-sectional view of the first flywheel of FIG. 5.
FIG. 7 is an enlarged view of a portion B shown in FIG. 6.
FIG. 8 is a perspective view of a first shaft sleeve of FIG. 2.
FIG. 9 is a cross-sectional view of the first shaft sleeve of FIG. 8.
FIG. 10 is a perspective view of a fixing base of the blood pump shown in FIG. 2.
FIG. 11 is a partial enlarged view of the blood pump shown in FIG. 2.
FIG. 12 is a perspective view of a second shaft sleeve of the blood pump shown in FIG. 2.
FIG. 13 is a perspective view of a stator and a magnetic conduction member of the blood pump shown in FIG. 1 assembled together.

### DETAILED DESCRIPTION OF THE EMBODIMENTS

In order to make the objectives, technical solutions, and advantages of the present application more clearly understood, the present application is described in further detail below in conjunction with the accompanying drawings and embodiments. It should be understood that specific embodiments described herein are intended only to interpret the present application and not intended to limit the present application.

It should be noted that when one element is referred to as "fixed to" or "arranged on" another element, it may be directly disposed on the other element or an intermediate element may exist. When one element is considered to be "connected to" another element, it may be directly connected to the other element or indirectly connected to the another element.

In addition, the terms "first" and "second" are used for descriptive purposes only, which cannot be construed as indicating or implying a relative importance, or implicitly specifying the number of the indicated technical features. Thus, the features defined with "first" and "second" may explicitly or implicitly include one or more features. In the description of the present application, "a plurality of' means two or more, unless specifically stated otherwise.

In order to illustrate the technical solutions of the present application, description will be made below with reference to specific drawings and embodiments.

In the field of interventional medicine, an end of a device adjacent to an operator is usually defined as a proximal end, and an end of the device away from the operator is defined as a distal end.

A blood pump 1 and a driving device 10 according to an embodiment of the present application are now described.

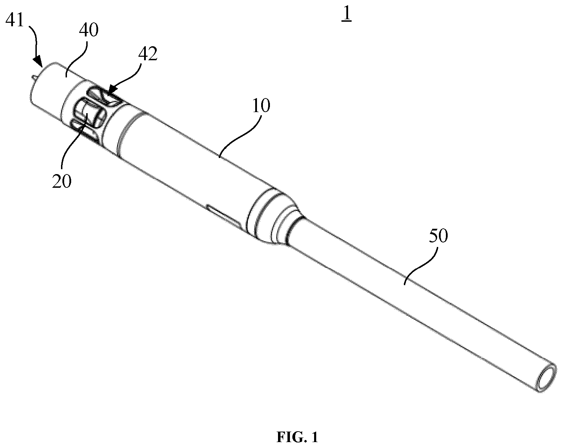

Referring to FIG. 1, the blood pump 1 includes a driving device 10 and an impeller 20. The driving device 10 is in transmission connection with the impeller 20, and the driving device 10 is capable of driving the impeller 20 to rotate.

Specifically, the blood pump 1 further includes a cannula 40 secured to a distal end of the driving device 10. The impeller 20 is rotatably accommodated in the cannula 40. The cannula 40 has a blood inlet 41 and a blood outlet 42. When the impeller 20 rotates, blood flows from the blood inlet 41 into the cannula 40 and then flows out of the blood outlet 42. In an embodiment, the cannula 40 extends through a heart valve, such as an aortic valve. The blood inlet 41 is located within a heart, and the blood outlet 42 and the driving device 10 are located in a blood vessel, such as an aorta outside a heart.

Specifically, the blood pump 1 further includes a catheter 50 connected to a proximal end of the driving device 10. The catheter 50 is configured to accommodate various supply lines. For example, the supply lines include a wire configured to be electrically connected to the driving device 10 and a cleaning pipeline configured to supply flushing fluid to the blood pump 1. Optionally, the flushing fluid is physiological saline, physiological saline containing heparin, or glucose, etc.

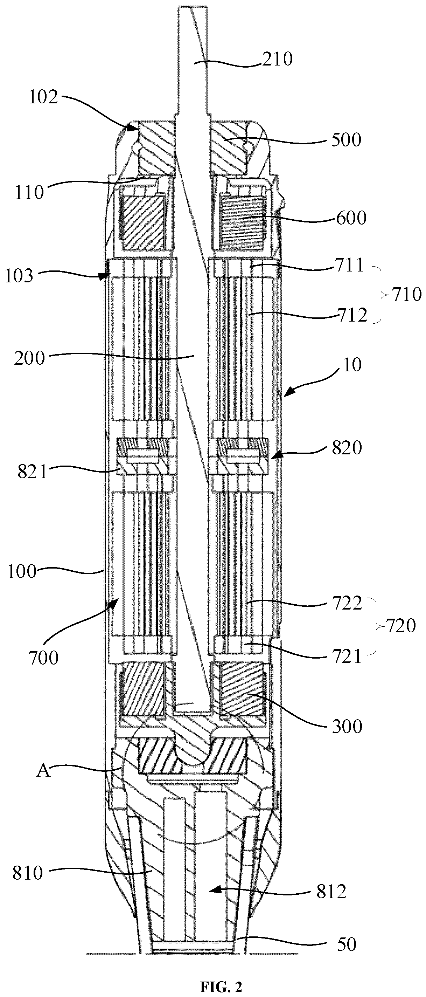

Referring to FIG. 2 to FIG. 4, the driving device 10 includes a housing 100, a rotating shaft 200, a first rotor 300, a first shaft sleeve 400, a second shaft sleeve 500, and a limiting member 600.

The housing 100 is substantially a cylindrical housing with openings at both ends thereof. A distal end of the housing 100 is secured to the cannula 40, and a proximal end of the housing 100 is secured to the catheter 50. The housing 100 has an inner cavity. Specifically, a separating ring 110 is provided in the housing 100, and the separating ring 110 divides the inner cavity of the housing 100 into a limiting cavity 102 and an accommodating cavity 103. In the illustrated embodiment, the limiting cavity 102 and the accommodating cavity 103 are arranged along an axial direction of the housing 100.

The rotating shaft 200 is elongated. The rotating shaft 200 is rotatably mounted to the housing 100, and the rotating shaft 200 is configured to be connected to the impeller 20. In the illustrated embodiment, the rotating shaft 200 extends substantially along the axial direction of the housing 100, in other words, an axis of the rotating shaft 200 extends in a direction substantially consistent with the axial direction of the housing 100. Therefore, the limiting cavity 102 and the accommodating cavity 103 are arranged substantially along the axis of the rotating shaft 200. The rotating shaft 200 extends through the limiting cavity 102, is partially accommodated in the accommodating cavity 103, and is partially located outside the housing 100 or extends partially into the cannula 40. Specifically, an end of the rotating shaft 200 configured to be connected to the impeller 20 is a connecting end 210.

In some embodiments, the rotating shaft 200 is made of a ceramic material. Compared with metal materials, ceramics have higher processing precision, higher biocompatibility and mechanical strength, and better wear resistance and corrosion resistance.

The first rotor 300 is fixedly connected to an end of the rotating shaft 200. Specifically, the first rotor 300 is fixedly connected to an end of the rotating shaft 200 away from the connecting end 210. The first rotor 300 has a spherical head portion 310 that protrudes along the axis of the rotating shaft 200 in a direction away from the connecting end 210. In the illustrated embodiment, the first rotor 300 is located in the housing 100. The first rotor 300 is located in the accommodating cavity 103. The first rotor 300 can rotate relative to the housing 100 and can drive the rotating shaft 200 to rotate.

Specifically, the driving device 10 further includes a stator 700 capable of driving the first rotor 300 to rotate. In the illustrated embodiment, the stator 700 is fixedly mounted in the housing 100, the stator 700 is specifically located in the accommodating cavity 103, and the rotating shaft 200 rotatably extends through the stator 700. In an embodiment, the first rotor 300 is magnetic, and the stator 700 is capable of generating a rotating magnetic field that drives the first rotor 300 to rotate.

Specifically, the first rotor 300 includes a first flywheel 320 fixedly connected to the rotating shaft 200 and a first magnet 330 fixedly connected to the first flywheel 320. The first magnet 330 and the first flywheel 320 cooperatively constitute a rotor body of the first rotor 300. The first magnet 330 is an annular Halbach array magnet. Specifically, the spherical head portion 310 is provided on the first flywheel 320. By providing the first flywheel 320, the connection strength between the first magnet 330 and the rotating shaft 200 can be enhanced, and the shaking of the rotating shaft 200 during rotation can be reduced, so that the whole rotating shaft 200 is more stable during rotation.

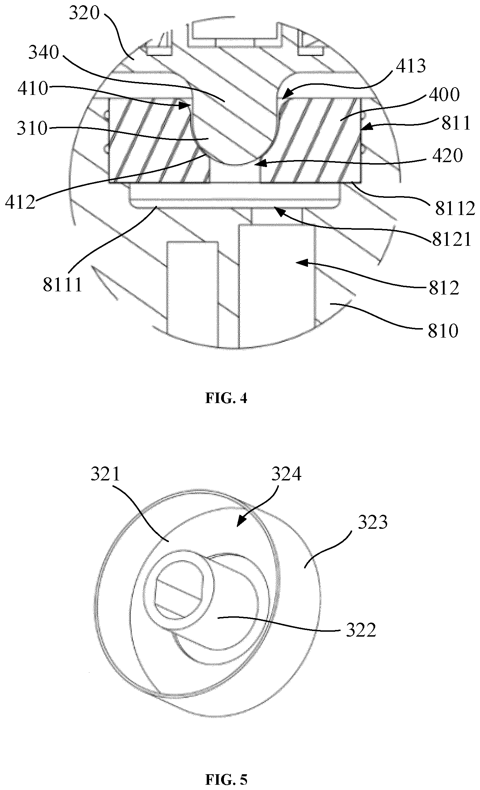

Specifically, referring to FIG. 5, the first flywheel 320 includes a first disc-shaped portion 321, a first inner tube 322, and a first outer tube 323. Both the first inner tube 322 and the first outer tube 323 are of circular tubular structures, and the first disc-shaped portion 321 is of an annular disc structure. Ends of the first inner tube 322 and the first outer tube 323 are fixedly connected to the first disc-shaped portion 321. The first inner tube 322 and the first outer tube 323 are located on the same side of the first disc-shaped portion 321 and are coaxially arranged. An inner diameter of the first outer tube 323 is greater than an outer diameter of the first inner tube 322. The first inner tube 322 is at least partially accommodated in the first outer tube 323. A first annular cavity 324 accommodating the first magnet 330 is formed between the first outer tube 323 and the first inner tube 322. A shape of the first annular cavity 324 is adapted to a shape of the first magnet 330, so as to facilitate mounting and positioning the first magnet 330. Such arrangement enables the first flywheel 320 to limit the first magnet 330, which not only facilitates the mounting of the first magnet 330, but also enables the first magnet 330 and the first flywheel 320 to be combined more stably.

Specifically, the end of the rotating shaft 200 away from the connecting end 210 is fixedly accommodated in the first inner tube 322, that is, the rotating shaft 200 does not extend through the first disc-shaped portion 321, so that the driving device 10 can be designed to be shorter. The spherical head portion 310 is located on a side of the first disc-shaped portion 321 away from the first inner tube 322.

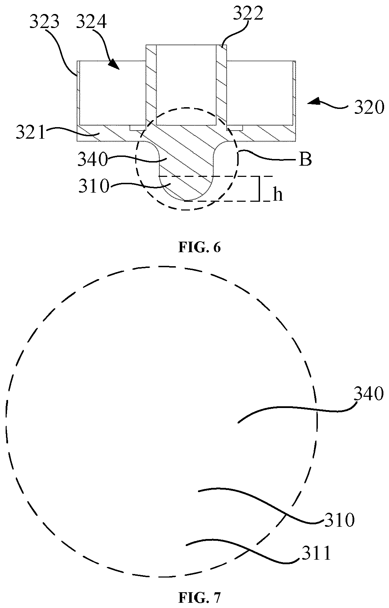

Referring to FIG. 6 and FIG. 7, specifically, the first rotor 300 further includes a connecting column portion 340 having one end fixedly connected to the rotor body, and the spherical head portion 310 is formed at an end of the connecting column portion 340 away from the rotor body. In other words, the end of the connecting pillar portion 340 away from the spherical head portion 310 is fixedly connected to the first flywheel 320 (specifically, the first disc-shaped portion 321). An axis of the connecting column portion 340 coincides with the axis of the rotating shaft 200. In the illustrated embodiment, the spherical head portion 310 is substantially hemispherical. The spherical head portion 310 has a spherical crown surface 311 and a circular bottom surface connected to the spherical crown surface 311. The circular bottom surface is connected to an end surface of the connecting column portion 340. The connecting column portion 340 is coaxial with the spherical head portion 310, and a diameter of an end surface of the connecting column portion 340 adjacent to the spherical head portion 310 is equal to a diameter of the circular bottom surface.

It should be noted that the first flywheel 320 is not limited to the above structure. In some embodiments, the first flywheel 320 does not have the first outer tube 323. In some embodiments, the first flywheel 320 does not have the first outer tube 323 and the first inner tube 322. In this case, the rotating shaft 200 fixedly extends through a center of the first disc-shaped portion 321. Compared to the first flywheel 320 having only the first disc-shaped portion 321, the first inner tube 322 enables the first flywheel 320 to be more stably connected to the rotating shaft 200.

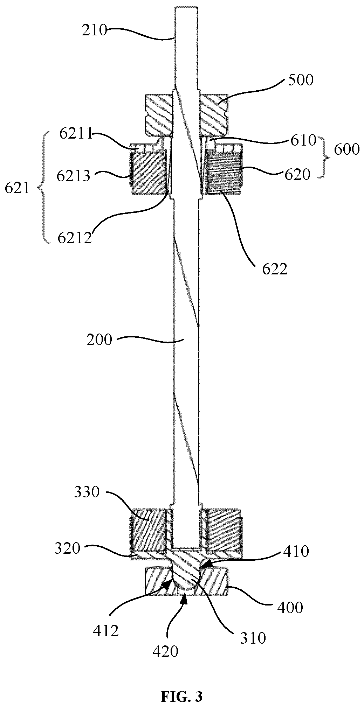

Referring to FIG. 2 and FIG. 3, both the first shaft sleeve 400 and the second shaft sleeve 500 are mounted to the housing 100. Specifically, the first shaft sleeve 400 is accommodated in the accommodating cavity 103, and the second shaft sleeve 500 is accommodated in the limiting cavity 102. Both the first shaft sleeve 400 and the second shaft sleeve 500 are fixedly connected to the housing 100. The first shaft sleeve 400 and the second shaft sleeve 500 are spaced apart from each other along the axial direction of the housing 100. The rotating shaft 200 rotatably extends through the second shaft sleeve 500, and the second shaft sleeve 500 is provided closer to the connecting end 210 of the rotating shaft 200 than the first shaft sleeve 400. The first rotor 300 is located between the first shaft sleeve 400 and the second shaft sleeve 500. The stator 700 is also located between the first shaft sleeve 400 and the second shaft sleeve 500.

Specifically, the first shaft sleeve 400 is provided with a groove 410 having a concave spherical groove wall 412. The spherical head portion 310 of the first rotor 300 is movably provided in the groove 410, and the spherical head portion 310 can abut against the spherical groove wall 412. The groove 410 can support and limit the spherical head portion 310 of the first rotor 300, so as to limit a movement range of the first rotor 300 and the rotating shaft 200 along the axis of the rotating shaft 200 in a direction away from the impeller 20, and at the same time, to limit a swinging range of the rotating shaft 200 in a radial direction of the rotating shaft 200.

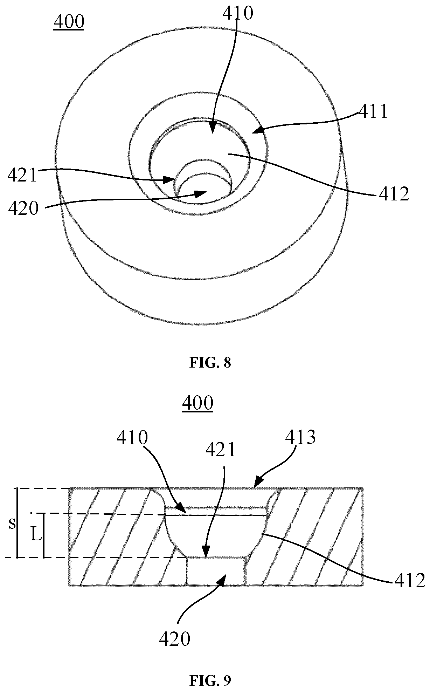

Referring to FIG. 8 and FIG. 9, specifically, the connecting column portion 340 is partially accommodated in the groove 410. The groove 410 has a notch 413, and the connecting column portion 340 extends through the notch 413. A length h of the spherical head portion 310 in an axial direction of the rotating shaft 200 is less than a depth s of the groove 410 (the depth s of the groove 410 is the maximum distance between the notch 413 of the groove 410 and the spherical groove wall 412), so as to better limit the spherical head portion 310 in the groove 410, and reduce radial swing ranges of the first rotor 300 and the rotating shaft 200. In the illustrated embodiment, a radius of the spherical groove wall 412 is greater than a radius of the spherical head portion 310, i.e., a radius of a sphere in which the spherical groove wall 412 is located is greater than a radius of a sphere in which the spherical head portion 310 is located. A length L of the spherical groove wall 412 in an axial direction of the first shaft sleeve 400 is less than the depth s of the groove 410.

Specifically, the notch 413 of the groove 410 is rounded, i.e., a groove wall at the notch 413 of the groove 410 is rounded, so as to prevent the connecting column portion 340 from being scratched and worn by the notch 413 of the groove 410 with the angular notch 413 of the groove 410.

Specifically, the spherical head portion 310 is provided with a diamond coating to make its surface smooth and improve wear resistance.

Specifically, the first shaft sleeve 400 is further provided with a liquid feeding hole 420 in communication with the groove 410. The liquid feeding hole 420 can be in fluid communication with the cleaning pipeline in the catheter 50, so that the flushing liquid can enter the groove 410 through the liquid feeding hole 420. The flushing liquid enters between the groove wall of the groove 410 and the spherical head portion 310 to serve as a lubricant, so as to reduce the friction between the spherical head portion 310 and the groove wall of the groove 410, thereby reducing the wear of the spherical head portion 310 and the first shaft sleeve 400.

Specifically, an opening 421 of the liquid feeding hole 420 is located at a center of the spherical groove wall 412, so that the flushing fluid entering the groove 410 from the liquid feeding hole 420 can provide an axial impact force to the spherical head portion 310 as much as possible. More specifically, a central axis of the liquid feeding hole 420 coincides with a central axis of a cavity enclosed by the spherical groove wall 412, so that the liquid feeding hole 420 is a straight hole to reduce the energy consumption of the flushing liquid in the liquid feeding hole 420.

Specifically, a diameter of the opening 421 of the liquid feeding hole 420 located on the spherical groove wall 412 is 1/9 to 1/3 of a diameter of a sphere where the spherical head portion 310 is located. In the illustrated embodiment, since the diameter of the liquid feeding hole 420 is constant, that is, the diameter of the liquid feeding hole 420 is 1/9 to 1/3 of the diameter of the sphere where the spherical head portion 310 is located. If the diameter of the opening 421 of the liquid feeding hole 420 on the spherical groove wall 412 is too large, a contact surface between the spherical head portion 310 and the spherical groove wall 412 will be reduced (resulting in a larger pressure per unit area), and the wear of the spherical head portion 310 by the spherical groove wall 412 will be increased. If the diameter of the opening 421 is too small, the amount of the flushing liquid entering the groove 410 from the liquid feeding hole 420 will be affected. On the one hand, the flushing liquid entering the groove 410 provides the spherical head portion 310 with the impact force, and at the same time, the flushing liquid enters between the spherical head portion 310 and the spherical groove wall 412 to serve as a lubricant, so as to reduce a friction coefficient between the spherical head portion 310 and the spherical groove wall 412. Therefore, the amount of the flushing liquid entering the groove 410 should not be too small.

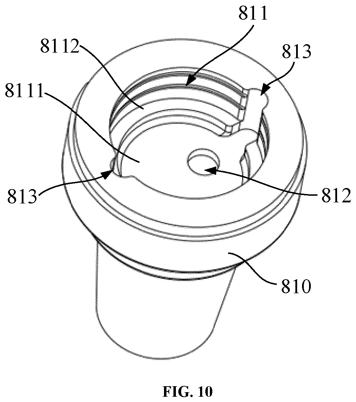

Referring to FIG. 2, FIG. 4, and FIG. 10, specifically, the driving device 10 further includes a fixing base 810 fixedly connected to the housing 100. The fixing base 810 is provided with a mounting cavity 811 and a liquid inlet hole 812 in communication with the mounting cavity 811. The first shaft sleeve 400 is mounted in the mounting cavity 811. The liquid feeding hole 420 is in communication with the liquid inlet hole 812. An end of the liquid inlet hole 812 away from the mounting cavity 811 is configured to be in communication with the cleaning pipeline of the catheter 50, so that the flushing liquid can flow between the groove wall of the groove 410 and the spherical head portion 310 through the liquid inlet hole 812 and the liquid feeding hole 420, and then flow into the inner cavity of the housing 100.

Specifically, the mounting cavity 811 has a cavity bottom 8111, and an opening 8121 (see FIG. 4) of the liquid inlet hole 812 is located at the cavity bottom 8111 of the mounting cavity 811. A supporting step 8112 is provided in the mounting cavity 811, and the supporting step 8112 abuts against the first shaft sleeve 400, so that the first shaft sleeve 400 is spaced from the cavity bottom 8111 by a certain distance, so as to better ensure the smooth circulation of the flushing liquid. Specifically, the supporting step 8112 abuts against a surface of the first shaft sleeve 400 away from the second shaft sleeve 500.

Specifically, the fixing base 810 is further provided with a branch channel 813, and the branch channel 813 is in fluid communication with the liquid inlet hole 812, so that fluid (such as flushing liquid) flowing through the liquid inlet hole 812 can also flow into the housing 100 through the branch channel 813. Specifically, one end of the branch channel 813 is in communication with a gap between the first shaft sleeve 400 and the cavity bottom 8111 of the mounting cavity 811, and the other end of the branch channel 813 is in communication with the accommodating cavity 103. In the illustrated embodiment, the branch channel 813 is formed by partially recessing a cavity wall of the mounting cavity 811. In other words, in a normal state, the flushing liquid is divided into two streams after entering the mounting cavity 811 from the liquid inlet hole 812, one stream flows into the groove 410 of the first shaft sleeve 400 through the liquid feeding hole 420, and the other stream flows out through the branch channel 813. The branch channel 813 can ensure the flow of the flushing liquid when the spherical head portion 310 blocks the liquid feeding hole 420.

In the illustrated embodiment, two branch channels 813 are provided, and the two branch channels 813 are arranged opposite to each other. It should be understood that the number of the branch channel 813 can be adjusted according to design requirements, for example, in some embodiments, one or more than two branch channels 813 are provided.

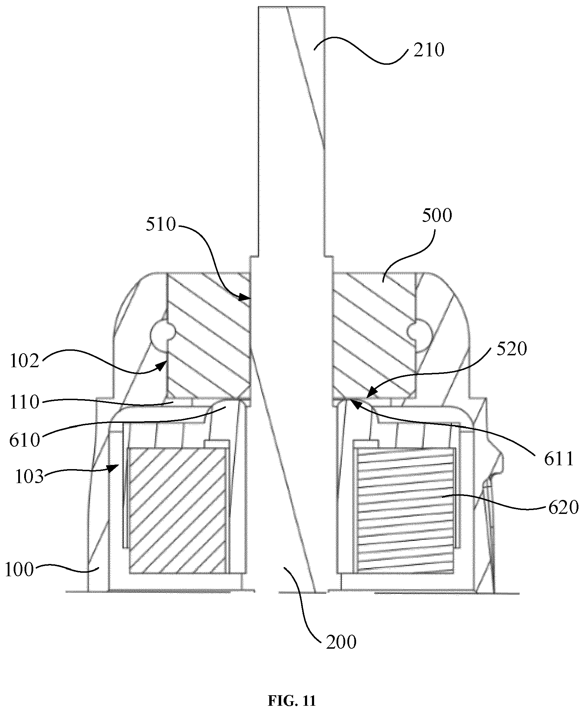

Referring to FIG. 2 and FIG. 11, the second shaft sleeve 500 abuts against the separating ring 110. In the illustrated embodiment, the second shaft sleeve 500 is accommodated in the limiting cavity 102, and the second shaft sleeve 500 can be conveniently positioned by the separating ring 110, so that the assembly of the second shaft sleeve 500 can be facilitated. The second shaft sleeve 500 is provided with a shaft hole 510, and the rotating shaft 200 rotatably extends through the shaft hole 510. In the illustrated embodiment, a central axis of the shaft hole 510 coincides with the central axis of the liquid feeding hole 420 of the first shaft sleeve 400. A gap is provided between a hole wall of the shaft hole 510 of the second shaft sleeve 500 and the rotating shaft 200 for the fluid to flow through. The flushing liquid entering the accommodating cavity 103 can flow out of the housing 100 through the gap between the rotating shaft 200 and the hole wall of the shaft hole 510.

The limiting member 600 is fixedly connected to the rotating shaft 200, the limiting member 600 is located between the second shaft sleeve 500 and the first rotor 300, and the limiting member 600 can abut against the second shaft sleeve 500, so as to limit a moving range of the rotating shaft 200 along the axis of the rotating shaft 200 in a direction approaching the second shaft sleeve 500. In the illustrated embodiment, the stator 700 is located between the first rotor 300 and the stop member 600.

Referring to FIG. 2, FIG. 3 and FIG. 11, the limiting member 600 includes a pushing stop ring 610 and a second rotor 620. The second rotor 620 is fixedly connected to the rotating shaft 200, and the pushing stop ring 610 is fixedly connected to at least one of the second rotor 620 and the rotating shaft 200. In other words, the pushing stop ring 610 may be directly fixed only to the second rotor 620, may be directly fixed only to the rotating shaft 200, or may be directly fixed to both the second rotor 620 and the rotating shaft 200. Since the second rotor 620 is fixedly connected to the rotating shaft 200, and the pushing stop ring 610 is fixedly connected to at least one of the second rotor 620 and the rotating shaft 200, the pushing stop ring 610, the rotating shaft 200, and the second rotor 620 can rotate and move synchronously. The pushing stop ring 610 is located between the second rotor 620 and the second shaft sleeve 500, and the pushing stop ring 610 can abut against the second shaft sleeve 500 to limit the movement range of the rotating shaft 200 along the axial direction of the rotating shaft 200 in a direction approaching the impeller 20.

In addition, the first rotor 300 is fixedly connected to one end of the rotating shaft 200, and the spherical head portion 310 of the first rotor 300 is provided in the groove 410 of the first shaft sleeve 400 and can abut against the spherical groove wall 412 of the groove 410, so as to limit the moving range of the rotating shaft 200 along the axis of the rotating shaft 200 in the direction away from the impeller 20, thereby limiting a position of the rotating shaft 200 on the axis of the rotating shaft 200. At the same time, since the rotating shaft 200 extends through the second shaft sleeve 500, and the spherical head portion 310 is provided in the groove 410 of the first shaft sleeve 400, the groove wall of the groove 410 of the first shaft sleeve 400 can also limit the swinging range of the spherical head portion 310 in the radial direction of the rotating shaft 200, thereby achieving the overall limitation of the swinging range of the rotating shaft 200 in the radial direction. In other words, the above design not only achieves an axial limit to the rotating shaft 200, but also achieves a radial limit to the rotating shaft 200.

The second rotor 620 is accommodated in the accommodating cavity 103, and the separating ring 110 is located between the second rotor 620 and the second shaft sleeve 500. Specifically, the second rotor 620 includes a second flywheel 621 fixedly connected to the rotating shaft 200 and a second magnet 622 fixed to the second flywheel 621. By arranging the second flywheel 621, the connection strength between the second magnet 622 and the rotating shaft 200 can be enhanced. In addition, the shaking of the rotating shaft 200 during rotation can be reduced, so that the whole rotating shaft 200 is more stable during rotation.

Optionally, the second magnet 622 is an annular Halbach array magnet.

Specifically, the second flywheel 621 includes a second disc-shaped portion 6211, a second inner tube 6212, and a second outer tube 6213. The second inner tube 6212 and the second outer tube 6213 are both of circular tubular structures, and the second disc-shaped portion 6211 is of an annular disc structure. Both the second inner tube 6212 and the second outer tube 6213 are fixedly connected to the second disc-shaped portion 6211. The second outer tube 6213 surrounds the second disc-shaped portion 6211, the second inner tube 6212 and the second outer tube 6213 are arranged coaxially, and the rotating shaft 200 extends through the second inner tube 6212 and is fixedly connected to the second inner tube 6212. A second annular cavity is formed between the second inner tube 6212 and the second outer tube 6213. The second magnet 622 is accommodated in the second annular cavity. A shape of the second annular cavity is adapted to the second magnet 622, so as to facilitate mounting and positioning the second magnet 622. Such arrangement enables the second flywheel 621 to limit the second magnet 622, which not only facilitates the mounting of the second magnet 622, but also enables the second magnet 622 and the second flywheel 621 to be combined more stably.

It should be noted that the second flywheel 621 is not limited to the above structure. In some embodiments, the second flywheel 621 does not have the second outer tube 6213. In some embodiments, the second flywheel 621 does not have the second inner tube 6212 and the second outer tube 6213, and at this time, the rotating shaft 200 fixedly extends through a center of the second disc-shaped portion 6211. Compared to the second flywheel 621 having only the second disc-shaped portion 6211, the second inner tube 6212 enables the second flywheel 621 to be more stably connected to the rotating shaft 200.

In the illustrated embodiment, the pushing stop ring 610 is an annular protrusion formed on a side of the second flywheel 621 away from the stator 700, and more specifically, the pushing stop ring 610 is provided on a side of the second disc-shaped portion 6211 away from the second inner tube 6212. That is, the pushing stop ring 610 and the second flywheel 621 are integrally formed as a whole. Since the overall volume of the blood pump 1 is small, the volume of the pushing stop ring 610 is smaller, it is difficult to be processed in precision, and has large assembly difficulty, the pushing stop ring 610 and the second flywheel 621 are integrally formed to facilitate the mounting, and the adhering operation is omitted.

It should be understood that in other embodiments, the pushing stop ring 610 and the second rotor 620 may also be of separate structures prior to assembly. In this case, the pushing stop ring 610 may be secured to at least one of the second rotor 620 and the rotating shaft 200 by adhering or welding.

Specifically, when the pushing stop ring 610 abuts against the second shaft sleeve 500, the pushing stop ring 610 is at least partially located in an inner ring of the separating ring 110, a gap for fluid to flow is provided between the pushing stop ring 610 and a wall of the inner ring of the separating ring 110, and the separating ring 110 is spaced apart from the second rotor 620 by a certain distance. By providing the gap for fluid to flow between the pushing stop ring 610 and the wall of the inner ring of the separating ring 110, the flushing liquid can flow into the shaft hole 510 of the second shaft sleeve 500 through the gap between the pushing stop ring 610 and the wall of the inner ring of the separating ring 110, that is, the fluid communication between the shaft hole 510 of the second shaft sleeve 500 and the accommodating cavity 103 is achieved. When the pushing stop ring 610 abuts against the second shaft sleeve 500, the separating ring 110 is spaced apart from the second rotor 620 by the certain distance, so as to avoid wear due to the friction between the second rotor 620 and the separating ring 110 when the pushing stop ring 610 abuts against the second shaft sleeve 500.

Specifically, the pushing stop ring 610 is substantially ring-shaped, and a central axis of the pushing stop ring 610 coincides with the axis of the rotating shaft 200. An outer diameter of the pushing stop ring 610 is less than an inner diameter of the separating ring 110, so that there is the gap for fluid to flow between the pushing stop ring 610 and the wall of the inner ring of the separating ring 110. In other embodiments, the pushing stop ring 610 may also be formed by arranging a plurality of sector rings that are evenly spaced around the rotating shaft 200 for one circumference, or may be understood as being formed by arranging a plurality of sector rings that are circumferentially discretely arranged.

Specifically, a thickness of the pushing stop ring 610 along the axis of the rotating shaft 200 is greater than a thickness of the separating ring 110 along the axis of the rotating shaft 200, so that the separating ring 110 is spaced apart from the second rotor 620 by a certain distance when the pushing stop ring 610 abuts against the second shaft sleeve 500. It should be understood that, in some embodiments, the thickness of the pushing stop ring 610 along the axis of the rotating shaft 200 can be less than or equal to the thickness of the separating ring 110 along the axis of the rotating shaft 200. In this case, the second rotor 620 and the pushing stop ring 610 may be spaced apart by a certain distance along the axis of the rotating shaft 200, and the distance is sufficient to ensure that the separating ring 110 and the second rotor 620 are spaced apart by a certain distance when the pushing stop ring 610 abuts against the second shaft sleeve 500.

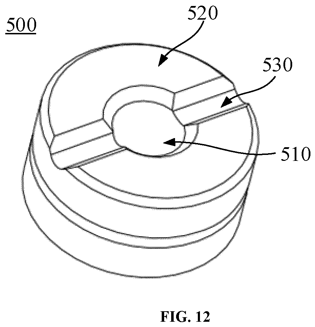

Specifically, referring to FIG. 12 together, a surface of the second shaft sleeve 500 facing the pushing stop ring 610 is partially recessed to form a guiding groove 530, and the guiding groove 530 is in communication with the shaft hole 510 of the second shaft sleeve 500. When the pushing stop ring 610 abuts against the second shaft sleeve 500, a part of the guiding groove 530 is not covered by the pushing stop ring 610, so that when the pushing stop ring 610 abuts against the second shaft sleeve 500, although the pushing stop ring 610 blocks the gap between the shaft hole 510 of the second shaft sleeve 500 and the rotating shaft 200, the guiding groove 530 not covered by the pushing stop ring 610 can achieve fluid communication when the pushing stop ring 610 abuts against the second shaft sleeve 500, so as to ensure the smoothness of the flushing liquid. In addition, the guiding groove 530 is formed by partially recessing the surface of the second shaft sleeve 500 facing the pushing stop ring 610, so that the flushing fluid can flow better between the pushing stop ring 610 and the second shaft sleeve 500 to lubricate the contact surface between the pushing stop ring 610 and the second shaft sleeve 500 and reduce the friction between the pushing stop ring 610 and the second shaft sleeve 500. The wear problem due to the friction between the pushing stop ring 610 and the second shaft sleeve 500 is reduced, and the heat dissipation effect on the pushing stop ring 610 and the second shaft sleeve 500 is achieved.

Specifically, the pushing stop ring 610 has a blocking surface 611 perpendicular to the axis of the rotating shaft 200. The second shaft sleeve 500 has a stopping surface 520 perpendicular to the central axis of the shaft hole 510 of the second shaft sleeve 500. The stopping surface 520 is opposite to the blocking surface 611, and the stopping surface 520 can abut against the blocking surface 611, so as to limit the movement of the rotating shaft 200 along the axis of the rotating shaft 200 in a direction approaching the impeller 20. Since the blocking surface 611 is perpendicular to the axis of the rotating shaft 200, and the stopping surface 520 is perpendicular to the central axis of the shaft hole 510 of the second shaft sleeve 500, the rotating shaft 200 rotatably extends through the shaft hole 510 of the second shaft sleeve 500, so that when the rotating shaft 200 operates normally and the pushing stop ring 610 abuts against the second shaft sleeve 500, the blocking surface 611 and the stopping surface 520 can be in contact with each other, which can reduce the wear caused by the friction between the pushing stop ring 610 and the second shaft sleeve 500. Specifically, the stopping surface 520 abuts against the separating ring 110. The guiding groove 530 is formed by partially recessing the stopping surface 520

Specifically, a roughness of at least one of the blocking surface 611 and the stopping surface 520 is less than or equal to 0.1 microns. In some embodiments, the roughness of both the blocking surface 611 and the stopping surface 520 is less than or equal to 0.1 microns. In some embodiments, the roughness of one of the blocking surface 611 and the stopping surface 520 is less than or equal to 0.1 microns. By reducing the roughness of at least one of the blocking surface 611 and the stopping surface 520, the friction between the blocking surface 611 and the stopping surface 520 can be effectively reduced, and the wear problem due to the friction between the second shaft sleeve 500 and the pushing stop ring 610 can be reduced.

In some embodiments, at least one of the blocking surface 611 and the stopping surface 520 is a ceramic surface. Ceramic has high machining accuracy, high biocompatibility, high mechanical strength, good wear resistance and corrosion resistance. In this case, the pushing stop ring 610 and the second shaft sleeve 500 may be made of ceramic, or at least one of the blocking surface 611 and the stopping surface 520 may be a ceramic surface by providing a ceramic coating. In some embodiments, the blocking surface 611 is made of diamond, so that the blocking surface 611 has a relatively high hardness, a relatively smooth surface, and is wear-resistant. In this case, the material of the blocking surface 611 is a ceramic surface by providing a diamond coating.

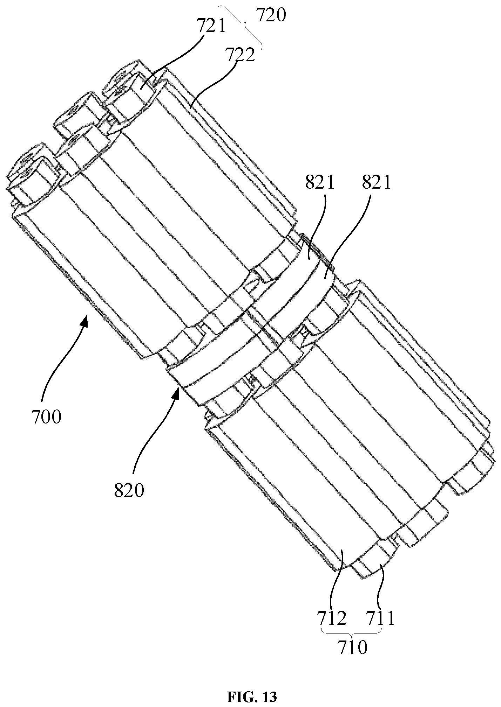

Specifically, referring to FIG. 2 and FIG. 13, the stator 700 includes a first stator unit 710 and a second stator unit 720 arranged along the axis of the rotating shaft 200. The first stator unit 710 is capable of driving the first rotor 300 to rotate, and the second stator unit 720 is capable of driving the second rotor 620 to rotate. Specifically, the first stator unit 710 can generate a rotating magnetic field to drive the first rotor 300 to rotate, and the second stator unit 720 can generate a rotating magnetic field to drive the second rotor 620 to rotate. Both the first stator unit 710 and the second stator unit 720 are fixedly accommodated in the accommodating cavity 103 of the housing 100. The rotating shaft 200 rotatably extends through the first stator unit 710 and the second stator unit 720. The first stator unit 710 and the second stator unit 720 are both located between the first rotor 300 and the second rotor 620. Specifically, the first rotor 300, the second rotor 620, the first stator unit 710, and the second stator unit 720 are located between the first shaft sleeve 400 and the second shaft sleeve 500. The first rotor 300 is adjacent to the first shaft sleeve 400, and the second rotor 620 is adjacent to the second shaft sleeve 500. In other words, the first shaft sleeve 400, the first rotor 300, the first stator unit 710, the second stator unit 720, the second rotor 620, and the second shaft sleeve 500 are arranged sequentially along the axis of the rotating shaft 200, and the second shaft sleeve 500 is located closest to the connecting end 210 of the rotating shaft 200.

The first stator unit 710 and the second stator unit 720 each includes a magnetic core and a coil wound around the magnetic core. Specifically, the first stator unit 710 includes a first magnetic core 711 and a first coil 712 wound around the first magnetic core 711. A plurality of first magnetic cores 711 are provided, and the plurality of first magnetic cores 711 are arranged around the axis of the rotating shaft 200 for one circumference. Each first magnetic core 711 corresponds to one first coil 712.

The structure of the second stator unit 720 is similar to that of the first stator unit 710. The second stator unit 720 includes a second magnetic core 721 and a second coil 722 wound around the second magnetic core 721. A plurality of second magnetic cores 721 are provided, and the plurality of second magnetic cores 721 are arranged around the axis of the rotating shaft 200 for one circumference. Each second magnetic core 721 corresponds to one second coil 722.

Specifically, the driving device 10 further includes a magnetic conduction member 820 fixedly connected to the housing 100. Both the first magnetic core 711 of the first stator unit 710 and the second magnetic core 721 of the second stator unit 720 are fixedly connected to the magnetic conduction member 820. Specifically, the magnetic conduction member 820 is fixedly accommodated in the housing 100, e.g., snapped into an inner side wall of the housing 100. The rotating shaft 200 rotatably extends through the magnetic conduction member 820. One end of the first magnetic core 711 is fixedly connected to the magnetic conduction member 820, and the first rotor 300 is adjacent to the other end of the first magnetic core 711. One end of the second magnetic core 721 is fixedly connected to the magnetic conduction member 820, and the second rotor 620 is adjacent to the other end of the second magnetic core 721.

The magnetic conduction member 820 serves to close a magnetic circuit, so as to promote and increase generation of the magnetic flux and improve a coupling capability. Therefore, the magnetic conduction member 820 can function to close the magnetic circuit between the first stator unit 710 and the first rotor 300, and to close the magnetic circuit between the second stator unit 720 and the second rotor 620, so as to increase the magnetic flux. Therefore, the arrangement of the magnetic conduction member 820 helps to reduce an overall diameter of the driving device 10. In addition, both the first magnetic core 711 of the first stator unit 710 and the second magnetic core 721 of the second stator unit 720 are fixedly connected to the magnetic conduction member 820, so that the first stator unit 710 and the second stator unit 720 can be positioned and mounted, and the assembly difficulty of the first stator unit 710 and the second stator unit 720 is reduced. At the same time, the magnetic conduction member 820 provided in the above manner can also reduce arrangement of a positioning structure in the housing 100, thereby simplifying the structure of the housing 100 and simplifying the assembly process of the entire driving device 10.

Specifically, the magnetic conduction member 820 includes two magnetic conduction plates 821 that are stacked. One magnetic conduction plate 821 is fixedly connected to the first magnetic core 711 of the first stator unit 710, and the other magnetic conduction plate 821 is fixedly connected to the second magnetic core 721 of the second stator unit 720. The rotating shaft 200 rotatably extends through the two magnetic conduction plates 821. Specifically, the two magnetic conduction plates 821 are separated prior to assembly. Since the magnetic conduction member 820 is configured as the two magnetic conduction plates 821 that are separated prior to assembly, when assembling the driving device 10, the first magnetic core 711 can be first fixedly connected to the magnetic conduction plate 821, the second magnetic core 721 can be fixedly connected to the other magnetic conduction plate 821, and then the two magnetic conduction plates 821 can be stacked. In this way, the first magnetic core 711 and the second magnetic core 721 can be easily assembled to the two magnetic conduction plates 821, respectively, and the first magnetic core 711 and the second magnetic core 721 can be assembled more easily.

Specifically, the two magnetic conduction plates 821 are fixedly connected, so that the first stator unit 710, the second stator unit 720, and the magnetic conduction member 820 are integrally formed and assembled into the housing 100, and the assembly of the stator 700 is easier. For example, the two magnetic conduction plates 821 may be connected together by gluing or welding. It should be understood that in other embodiments, the two magnetic conduction plates 821 are not fixedly connected, but are in contact with each other.

It should be noted that the magnetic conduction member 820 is not limited to the combination of the two magnetic conduction plates 821 that are separated, and the magnetic conduction member 820 may be a plate-like structure. Both the first magnetic core 711 and the second magnetic core 721 are connected to the magnetic conduction member 820, that is, the first stator unit 710 and the second stator unit 720 share one magnetic conduction member 820.

Specifically, the magnetic conduction plate 821 is made of silicon steel, and the first magnetic core 711 and the second magnetic core 721 are made of silicon steel.

The first magnetic core 711 and the second magnetic core 721 each has a columnar structure without a head portion (i.e., a pole shoe) having a large width. Compared with the magnetic core having the pole shoe, the magnetic core of the columnar structure can reduce the magnetic loss and increase the magnetic coupling density between the magnetic core and the magnet to increase the torque of the stator 700 to the magnet (under the same current conditions). In addition, the magnetic core without the head portion can also greatly reduce the problems of local magnetic short circuit and motor power reduction caused by the contact between adjacent magnetic cores.

It should be understood that the structure of the driving device 10 is not limited to the above structure. In some embodiments, the driving device 10 includes the first rotor 300, the second rotor 620, and the stator 700, but the stator 700 has only one stator unit, i.e., only the first stator unit 710, without the second stator unit 720. At this time, the first stator unit 710 is located between the first rotor 300 and the second rotor 620, and the first stator unit 710 is capable of simultaneously driving the first rotor 300 and the second rotor 620 to rotate.

It should be understood that the limiting member 600 is not limited to the above structure. In some embodiments, the limiting member 600 only has the pushing stop ring 610 without the second rotor 620. In this case, the stator 700 only has one stator unit. The pushing stop ring 610 is fixedly connected to the rotating shaft 200 and abuts against the second shaft sleeve 500, so as to limit the movement range of the rotating shaft 200 along the axis thereof in a direction approaching the second shaft sleeve 500. In some embodiments, the limiting member 600 is only the second rotor 620 without the pushing stop ring 610. At this time, the second rotor 620 is fixedly connected to the rotating shaft 200, the stator 700 is located between the first rotor 300 and the second rotor 620, and a side of the second rotor 620 away from the stator 700 abuts against the second shaft sleeve 500 or the separating ring 110, so as to limit the movement range of the rotating shaft 200 along the axis thereof in a direction approaching the impeller 20.

Since the driving device of this embodiment has a structure similar to that of the driving device of the first embodiment, the driving device of this embodiment and the blood pump having the driving device of this embodiment also have effects similar to those of the first embodiment.

The foregoing embodiments are merely intended to describe the technical solutions of the present application, but not to limit the present application. Although the present application is described in detail with reference to the foregoing embodiments, those of ordinary skill in the art should understand that they may still make modifications to the technical solutions described in the foregoing embodiments or make equivalent replacements to some technical features thereof, without departing from the spirit and scope of the technical solutions of the embodiments of the present application, all of which fall within the protection scope of the present application.

## Claims

1. A driving device, comprising:
a housing ;
a rotating shaft configured to be connected to an impeller, the rotating shaft being rotatably mounted to the housing;
a first rotor fixedly connected an end of the rotating shaft, the first rotor comprising a spherical head portion protruding along an axis of the rotating shaft;
a first shaft sleeve mounted to the housing, the first shaft sleeve being provided with a groove having a concave spherical groove wall, wherein the spherical head portion is movably provided in the groove, and the spherical head portion is capable of abutting against the spherical groove wall;
a second shaft sleeve mounted to the housing, wherein the rotating shaft rotatably extends through the second shaft sleeve, and the first rotor is located between the first shaft sleeve and the second shaft sleeve; and
a limiting member fixedly connected to the rotating shaft, the limiting member being located between the second shaft sleeve and the first rotor, and the limiting member being capable of abutting against the second shaft sleeve.

2. The driving device according to claim 1, wherein the first rotor comprises a first flywheel and a first magnet, the first flywheel is fixedly connected to the rotating shaft, the first magnet is fixedly connected to the first flywheel, the spherical head portion is provided on the first flywheel, the driving device further comprises a stator located between the first rotor and the limiting member, and the stator is capable of generating a rotating magnetic field that drives the first magnet to rotate.

3. The driving device according to claim 2, wherein the first flywheel comprises a first disc-shaped portion, a first inner tube, and a first outer tube, ends of the first inner tube and the first outer tube are fixedly connected to the first disc-shaped portion, the first inner tube and the first outer tube are located on a same side of the first disc-shaped portion and are coaxially arranged, an inner diameter of the first outer tube is greater than an outer diameter of the first inner tube, the first inner tube is at least partially accommodated in the first outer tube, a first annular cavity accommodating the first magnet is formed between the first outer tube and the first inner tube, the rotating shaft comprises a connecting end configured to be connected to the impeller, an end of the rotating shaft away from the connecting end is fixedly accommodated in the first inner tube, and the spherical head portion is located on a side of the first disc-shaped portion away from the first inner tube.

4. The driving device according to claim 1, wherein the first shaft sleeve is further provided with a liquid feeding hole in communication with the groove, the driving device further comprises a fixing base fixedly connected to the housing, the fixing base is provided with a mounting cavity and a liquid inlet hole in communication with the mounting cavity,, the first shaft sleeve is mounted in the mounting cavity, and the liquid feeding hole is in fluid communication with the liquid inlet hole.

5. The driving device according to claim 4, wherein the mounting cavity has a cavity bottom, an opening of the liquid inlet hole is located at the cavity bottom, a supporting step is provided in the mounting cavity, and the supporting step abuts against the first shaft sleeve, so that the first shaft sleeve is spaced from the cavity bottom by a certain distance;
and/or the fixing base is further provided with a branch channel in communication with the liquid inlet hole, so that fluid entering the liquid inlet hole is further capable of flowing into the housing through the branch channel;
and/or an opening of the liquid feeding hole is located on the spherical groove wall, the opening is located at a center of the spherical groove wall, and a diameter of the opening is 1/9 to 1/3 of a diameter of a sphere where the spherical head portion is located.

6. The driving device according to claim 4, wherein a notch of the groove is rounded, and the spherical head portion is provided with a diamond coating.

7. The driving device according to claim 1, wherein the first rotor further comprises a rotor body and a connecting column portion having one end fixedly connected to the rotor body, the rotor body is fixedly connected to the rotating shaft, the spherical head portion is formed at one end of the connecting column portion away from the rotor body, a length of the spherical head portion in an axial direction of the rotating shaft is less than a depth of the groove, and the connecting column portion is partially accommodated in the groove.

8. The driving device according to claim 7, wherein the spherical head portion is hemispherical, the spherical head portion comprises a spherical crown surface and a circular bottom surface connected to the spherical crown surface, the circular bottom surface is connected to an end surface of the connecting column portion, the connecting column portion is coaxial with the spherical head portion and a diameter of an end surface of the connecting column portion adj acent to the spherical head portion is equal to a diameter of the circular bottom surface.

9. The driving device according to claim 1, wherein the limiting member comprises a second rotor and a pushing stop ring, the second rotor is fixedly connected to the rotating shaft, the pushing stop ring is fixedly connected to at least one of the second rotor and the rotating shaft, and the pushing stop ring is capable of abutting against the second shaft sleeve.

10. The driving device according to claim 9, wherein the second rotor comprises a second flywheel and a second magnet, the second flywheel is fixedly connected to the rotating shaft, the second magnet is fixedly connected to the second flywheel, the driving device further comprises a stator located between the first rotor and the second rotor, the stator is capable of generating a rotating magnetic field that drives the second magnet to rotate, and the pushing stop ring is an annular protrusion formed on a side of the second flywheel away from the stator.

11. The driving device according to claim 9, wherein a separating ring is provided in the housing, the separating ring divides an inner cavity of the housing into a limiting cavity and an accommodating cavity, the separating ring is located between the second shaft sleeve and the second rotor, the second shaft sleeve is accommodated in the limiting cavity and abuts against the separating ring, and the second rotor is accommodated in the accommodating cavity;
when the pushing stop ring abuts against the second shaft sleeve, the pushing stop ring is at least partially located in an inner ring of the separating ring, a gap for fluid to flow is provided between the pushing stop ring and a wall of the inner ring of the separating ring, and the separating ring is spaced apart from the second rotor by a certain distance.

12. The driving device according to claim 11, wherein a thickness of the pushing stop ring along the axis of the rotating shaft is greater than a thickness of the separating ring along the axis of the rotating shaft, so that when the pushing stop ring abuts against the second shaft sleeve, the separating ring is spaced apart from the second rotor by the certain distance.

13. The driving device according to claim 9, wherein the pushing stop ring comprises a blocking surface perpendicular to the axis of the rotating shaft, the second shaft sleeve comprises a stopping surface opposite to the blocking surface, the stopping surface is capable of abutting against the blocking surface, the stopping surface is provided with a guiding groove in communicated with a shaft hole of the second shaft sleeve to allow flushing liquid to flow, and when the stopping surface abuts against the blocking surface, a part of the guiding groove is not covered by the pushing stop ring.

14. The driving device according to claim 13, wherein a roughness of at least one of the blocking surface and the stopping surface is less than or equal to 0.1 microns, or at least one of the blocking surface and the stopping surface is a ceramic surface.

15. The driving device according to claim 9, wherein the pushing stop ring is ring-shaped, a central axis of the pushing stop ring coincides with the axis of the rotating shaft; or the pushing stop ring is formed by arranging a plurality of sector rings that are arranged evenly spaced around the rotating shaft for one circumference.

16. The driving device according to claim 10, wherein the pushing stop ring and the second flywheel of the second rotor are integrally formed; or the pushing stop ring is fixed to at least one of the second rotor and the rotating shaft by adhering or welding.

17. The driving device according to claim 9, further comprising a stator, wherein the stator comprises a first stator unit and a second stator unit arranged along the axis of the rotating shaft, both the first stator unit and the second stator unit are located between the first rotor and the second rotor, the first stator unit is capable of driving the first rotor to rotate, and the second stator unit is capable of driving the second rotor to rotate.

18. The driving device according to claim 17, wherein the first stator unit comprises a first magnetic core and a first coil wound around the first magnetic core, the second stator unit comprises a second magnetic core and a second coil wound around the second magnetic core, the driving device further comprises a magnetic conduction member fixedly connected to the housing, and the magnetic conduction member is fixedly connected to both the first magnetic core and the second magnetic core.

19. The driving device according to claim 18, wherein the magnetic conduction member comprises two magnetic conduction plates that are stacked, one magnetic conduction plate is fixedly connected to the first magnetic core, and another magnetic conduction plate is fixedly connected to the second magnetic core.

20. A blood pump, comprising an impeller and a driving device, wherein the driving device comprises:
a housing;
a rotating shaft configured to be connected to an impeller, the rotating shaft being rotatably mounted to the housing;
a first rotor fixedly connected an end of the rotating shaft, the first rotor comprising a spherical head portion protruding along an axis of the rotating shaft;
a first shaft sleeve mounted to the housing, the first shaft sleeve being provided with a groove having a concave spherical groove wall, wherein the spherical head portion is movably provided in the groove, and the spherical head portion is capable of abutting against the spherical groove wall;
a second shaft sleeve mounted to the housing, wherein the rotating shaft rotatably extends through the second shaft sleeve, and the first rotor is located between the first shaft sleeve and the second shaft sleeve; and
a limiting member fixedly connected to the rotating shaft, the limiting member being located between the second shaft sleeve and the first rotor, and the limiting member being capable of abutting against the second shaft sleeve;
wherein the rotating shaft is connected to the impeller, and the rotating shaft is capable of driving the impeller to rotate.
